# EUROPEAN PATENT SPECIFICATION

(11) **EP 1 928 474 B1**
(45) Date of publication and mention of the grant of the patent: **03.10.2012**
(21) Application number: 06780614.1
(22) Date of filing: 11.08.2006
(51) Int. Cl.: A61K 31/7028, A61P 27/02

(54) **USE OF GLUCOSYLATED HYDROXYSTILBENES FOR THE PREVENTION AND TREATMENT OF EYE PATHOLOGIES**
VERWENDUNG VON GLUCOSYLIERTEN HYDROXYSTILBENEN ZUR PRÄVENTION UND BEHANDLUNG VON AUGENERKRANKUNGEN
UTILISATION DE L'HYDROXYSTILBENES GLUCOSYLES POUR LA PREVENTION ET LE TRAITEMENT DE PATHOLOGIES OCULAIRES

(30) Priority: 19.08.2005 IT RM20050446
(43) Date of publication of application: 11.06.2008
(73) Proprietor: Tubilux Pharma S.P.A., I-00040 Pomezia (IT)
(72) Inventor: FALCHETTI, Roberto, I-00040 Pomezia (IT)
(74) Representative: Banchetti, Marina
(86) International application number: PCT/IT2006/000621
(87) International publication number: WO 2007/020673

(56) References cited:
- EP-A2- 0 773 020
- WO-A2-00/12534
- US-A1- 2005 096 256

## Description

The present invention concerns the use of glucosylated hydroxystil-benes for the prevention and treatment of eye pathologies. More specifically, the invention concerns the use of compounds of natural origin that are extractable from the tissues of some plant species (but are also obtainable via chemical synthesis), including, in particular, piceide or resveratrol-3-O-β-mono-D-glucoside, for the preparation of pharmaceutical and/or nutritional compositions that turned out to be effective upon oral, intravenous, intramuscular and ocular topical administration in preserving and restoring the structural and functional characteristics of eye tissues in various ophthalmic pathologies.

As is known, the eye is a hollow, fluid-filled organ divided into two segments by the crystalline lens. The anterior segment is delimited at the front by the cornea and at the back by the iris-body ciliar-lens complex. The resulting cavity is full of a transparent liquid, the aqueous humour, produced by ciliary processes. The posterior segment, which accounts for four-fifths of the volume of the entire eyeball, is delimited at the front by the crystalline lens and at the back by the sclera-choroid-retina complex. The resulting cavity is full of a transparent gel, the vitreous humour.

The retina is the nervous tissue responsible for converting light stimuli into electric signals. It has a complex structure made up of photoreceptors, bipolar cells, support cells and ganglion cells. The axons of the latter cells line the internal surface of the retina and are collected together into a sheath, the optic nerve, which is responsible for conveying the electric signal to the visual regions of the brain (geniculate bodies and visual cortex). The optic nerve, consisting of about one million axons, starts from the eyeball at the optic papilla.

Due to its structure, the various anatomical components of the eye (in particular, the cornea, lens and retina) are continually exposed to photooxidative reactions caused by light, and thus exposed to the harmful action of free radicals that are continuously generated inside the cells. Some free radicals that are more damaging to human body tissues derive from oxygen: as is known, the term "reactive oxygen species" (ROS) is used to collectively refer to the free radicals and non-radical chemical species that currently take part in oxidative biological processes and whose surplus with respect to natural equilibrium conditions is considered to be the cause of an increasing number of degenerative and pathological phenomena. More specifically, the term ROS includes the anionic superoxide radical, O₂, the hydroxy radical, OH·, the singleton oxygen, ¹O₂, hydrogen peroxide, H₂O₂, as well as the alcohol radicals, RO·, and peroxy radicals ROO·, which are formed by organic molecules during the oxidative processes.

Reactive oxygen species are, for example, produced by activated macrophagi and their release represents an important defence mechanism on the part of the host organism. However, due to their high reactivity, ROS can have important toxic effects on other molecules and can cause cell dysfunctions and sometimes even cell death. Therefore, their build-up in tissues can give rise to various pathologies, such as some types of neoplasy, cardiovascular diseases and neurodegenerative diseases. In particular, the results of a vast series of studies indicate that free radicals (specially the superoxide radical and hydroxy radicals) are mediators of acute and chronic inflammation.

In the body there is a set of molecular defence mechanisms against free radicals. In general, the molecules and processes referred to as antioxidants, which, as a whole, form an antioxidant defence system, directly neutralise the free radicals or serve to free the cells from ROS. Various agents naturally present in cell tissues can carry out the aforesaid activity, in practice acting as scavengers. Among these, the most known are the vitamins E (α-tocopherol) and C (ascorbic acid), the antioxidant enzymes like superoxydedismutase (SOD), catalase, peroxidase and glutathione reductase, and various substances of low molecular weight, including glutathione (GSH) and tyrosin. In physiological conditions, there is an equilibrium between the ROS levels produced during the normal cell metabolism and endogenous antioxidant levels, which protects the tissues from oxidative damage. The breakdown of this equilibrium, both due to an increase in ROS production and owing to a decrease in antioxidant levels, causes a condition generally known as oxidative stress, which can lead to the onset of different pathological conditions - also affecting the tissues of the visual apparatus.

The cornea, which is a transparent avascular tissue enabling the passage of incident light to the posterior parts of the eye, absorbs about 80% of the ultraviolet B (UV-B) light rays and is thus continuously exposed to oxidative stress. A healthy cornea normally possesses various defence mechanisms that can reduce the risk of damage from oxidative stress, such as the presence of an isoenzyme of aldehyde dehydrogenase (ALDH3) that can absorb UV rays and remove the aldehydes produced by the lipid peroxidation caused by the UV rays, or the presence of the aforesaid antioxidant enzymes, which can remove the free radicals generated following a continuous absorption of UV light. In the case of some pathologies of the cornea, however, these defences become undermined and thus oxidative stress can give rise to inflammatory processes and lead to cell death, through necrotic and apoptotic mechanisms.

Schematically, the damaging effects of oxidative stress can come about both through the hyperoxidation of lipids (lipoperoxidation) caused by an increase in ROS, with the resulting release of toxic substances, or by stimulating the release of inducible nitric-synthesase oxide (iNOS) on the part of the corneal cells, with the subsequent synthesis of NO. Nitrogen monoxide is an extremely reactive molecule which, in overproduction conditions, contributes to damaging the tissues. It also has the capacity to regulate the expression of inflammatory proteins, in particular, by increasing the expression of cyclooxigenase-2 (COX-2), an enzyme regulating the production of pro-inflammatory prostaglandin, and of TNF-α, a cytochin that is also pro-inflammatory.

A further effect of oxidative stress on the cornea, as well as on other ocular structures, is the induction of apoptosis (i.e. the programmed cell death process characterised by cell chromatin condensation, cell contraction, apoptotic body formation and cell DNA breakdown into numerous fragments), by altering the activity of the mitochondrial caspase and of Bcl₂, an anti-apoptotic protein situated in the mitochondria.

Oxidative stress can lead to acute corneal pathologies, such as photokeratitis, and pathologies from chronic exposure, such as pinguecula and pterigium. Moreover, it contributes to the development and/or aggravation of high incidence pathologies in the normal population, such as dry-eye syndrome.

Dry eye (also known as dry keratoconjunctivitis, from one of its more common manifestations) is a term that includes various very common eye pathologies affecting millions of people all over the world, particularly the female sex. There are many causes of dry eye, including, for example, certain systemic disorders (such as rheumatoid arthritis and lupus eritematosus), particular physiological conditions (such as menopause), eye diseases (such as blepharitis, pathologies of meibomian glands), environmental conditions (such as UV-B oxidative stress) or even the use of contact lenses and extensive use of computer screens.

The various pathological manifestations of dry-eye syndrome have some common denominators, such as epitheliopathy of the ocular surface, the hyperosmolarity of tear fluid, the instability of tear film, symptoms like burning sensations, reddening, foreign body sensations and eye dryness. These are mainly due to chronic inflammation affecting different structures of the eye surface, such as the cornea, conjunctiva, the accessorial lachrymal glands and meibomian glands. Subjects suffering from dry keratoconjunctivitis have been found to have the expression of specific markers of inflammation and of apoptosis in the epithelial cells of the ocular surface, thus indicating a strong correlation between the onset of inflammatory and apoptotic processes and the development of the pathology. Some researchers also hypothesise that corneal cell apoptosis may be caused by pro-apoptotic signals induced by the drying of the eye surface - a characteristic always linked to dry eye.

The most common therapies for patients with dry eye mainly envisage using artificial tears in order to restore the correct quantity and quality of tear fluid, but these treatments do not act on the pathological process underlying the disorder.

Considering the by now ascertained importance of inflammation in the onset and protraction of dry eye, it is deemed that more encouraging and lasting results can be obtained by using anti-inflammatory treatments. Linked to these, considerable importance should be given to therapies able to inhibit apoptotic processes affecting corneal cells.

Turning now to consider other aspects linked to eye pathologies, the preservation of cornea transparency is an essential requirement of reparatory physiological processes, both as regards the epithelium and the corneal stroma. This need is also important in operations of cornea reshaping for refraction purposes, in particular by using excimer laser techniques known as photorefractive keratectomy, or PRK, and LASIK (laser intrastromal keratomileusis), for correcting various forms of amethropy including, first and foremost, myopia. Although these treatments are a less traumatic alternative to conventional ophthalmic surgery, the reparative process after photoablation is not devoid of more or less transitory bothersome or debilitating drawbacks for the patient, such as corneal healing problems, subepithelial opacity known as "haze", which reduces visual efficiency as a result of the "light scattering" phenomenon and, in some cases, can lead to the regression of the refractive values obtained with the operation. The literature of the field appears unanimous in attributing, at least partly, the onset of these effects to the formation of free radicals and, in general, to ROS, which have been found to be a side effect of UV radiation and of the temperature increases created in the tissues concerned. It is also deemed that these effects can even be partly due to inflammatory processes following laser treatment and surgery.

A mechanism correlated to repair processes is, at an early stage of the process, the stimulation of some corneal fibroblasts to turn into myofibroblasts (Jester V.J. et al., Invest. Ophthalmol. Vis. Sci., 2003, 44, 1850-1858). The latter express α-actin of the smooth muscle type (α-SMA) and form a meshwork of cells capable of exerting a mechanical force through a contractile mechanism of the smooth muscle type, that acts giving rise to the contraction of the lesion and to the organisation of the matrix.

In some cases, the myofibroblasts can reproduce in greater amounts than those physiologically necessary, or can remain longer than necessary in the region to be repaired. When this happens, there is an incorrect repair of the lesion, with the presence on the cornea of fibrotic damage and areas of reduced transparency. Even this phenomenon has been correlated to oxidative stress conditions.

Recent studies indicate that apoptosis plays an important homeostatic role also in repair mechanisms of corneal epithelium damaged by erosion processes or by UV radiation, since it contributes to regulating the various cell populations. In this case, too, an alteration of the apoptotic process can cause an incorrect regeneration pattern and thus lead to an incorrect repair of the cornea.

The crystalline lens consists of a single layer of epithelial cells incorporating concentric layers of elongated fibrous cells, which make up the cortical layer and nucleus of the lens. These cells are not renewed and thus, in cases where the physiological antioxidant defence systems present are not completely efficient, the oxidative damage builds up over the years, leading to irreversible changes in the optical properties of the lens. The build-up of this damage may lead to cataract, a pathology correlated with age, that consists of a hazing of the lens, and is the most frequent cause of blindness in the world. Among the various factors involved in the ethiology of cataract, the one playing the most important role is oxidative stress, since it causes the oxidation and build up of proteins in the lens, in particular, of methionine, which is oxidised to methionine sulfoxide. In cataract, about 60% of the methionine linked to the membrane turns out to be oxidised.

Getting back to the cornea, the phacoemulsification that is carried out during cataract operations, by using high-energy ultrasounds, produces free radicals, which can cause damage to corneal endothelial cells. This mechanism, along with others (such as the release of pro-inflammatory substances), may be considered to be an important cause of non-renewable endothelial cell loss during a cataract operation.

Oxidative stress is also considered to be one of the most important causes of the onset of age-related macular degeneration (AMD), a pathology that can lead to serious impairments of eyesight. It comes about as a damage to the photoreceptors of the macula, accompanied by anatomical and functional alterations affecting the retinal pigment epithelium (RPE) and Bruch membrane.

The cells of the external retina, and particularly the external segment of photoreceptors, contain, in their membrane, high concentrations of polyunsaturated fatty acids (PUFAs), which are extremely susceptible to oxidation in the presence of oxygen or ROS. The first signs of senescence of the external retinal layer is the presence, in the RPE, of lipofuscin - a generic name indicating a heterogeneous group of aggregate lipids/proteins that accumulate in various types of cells and tissues. In the eye the most abundant substratum for the formation of lipofuscin accumulating in the RPE is represented by a non-degradable product deriving from phagocytosis of the photoreceptors of the external segment. This product is considered to be the result of the lipid peroxidation taking place due to self-oxidation or exposure to ROS because of continuous exposure to visible light (400-700 nm) and to high oxygen pressure (70 mm Hg).

A second site in the retina where free radicals can give rise to photosensibilisation correlated to AMD is the coriocapillary, according to a mechanism envisaging the photoactivation of precursors of the haemoglobin present in the blood of capillaries, with the subsequent generation of ROS which in turn can damage the RPE and Bruch membrane.

One of the most important complications of AMD is the neovascularization of the retina, caused by the retinal cells releasing the vascular growth factor called "vascular endothelial growth factor", VEGF. This factor is also important in the ethiology of another pathology: choroid neovascularization (CNV).

Other retinal pathologies that have been associated, at least in part, to oxidative stress are diabetic retinopathy and proliferative vitreoretinopathy (PVR), which is a proliferative variety of the former, characterised by the neovascularisation of the retina and of the optic disc, which can spread to the vitreous humour, the proliferation of fibrous tissue and retinal punctiform haemorrhages.

Moving on, finally, to a degenerative ophthalmic pathology which alone is responsible for at least 15% of cases of blindness in the elderly, namely, glaucoma, this pathology is considered to be caused by an increase in intraocular pressure in 90-95% of the cases. In the remaining 5-10% of the cases it is thought that the ethiology of the pathology involves neurotoxic factors, including the formation of nitrogen reactive species, particularly NO. Some researchers suggest that the presence of this factor has some importance also when glaucoma is caused by an increase in intraocular pressure.

There are a great many molecules with antioxidant activity present in nature, belonging to different chemical classes. Among the smaller molecules with antioxidant activity found in nature, some - such as α-tocopherol, ascorbic acid, carotenoids, coenzyme Q, vitamin A, certain flavonoids and polyphenols - have been widely experimented for their pharmacological and therapeutic activity.

The results of many studies have highlighted that polyphenols possess a series of physio-pharmacological properties through which they can have many therapeutic effects in man. For example, resveratrol, a polyphenol of natural origin belonging to the stilbene family (α,β-diphenylethylene or dibenzylidene, in the *cis*- or *trans-* form with respect to the double bond), and more precisely being a hydroxystilbene (3,4,5'-trihydroxystilbene, whose cis-and *trans-* forms are shown below), has been found to have anti-inflammatory, cardioprotective and chemoprotective properties.

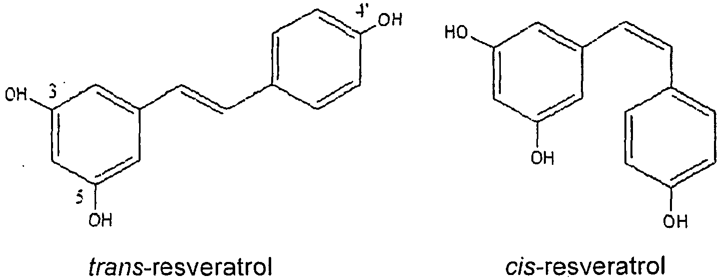

As is known, resveratrol and other related natural stilbene compounds have been included in the phytoalexins, a heterogeneous class of antibiotics of plant origin, because their role in plant physiology appears to be mainly that of inhibiting the spreading of fungine infections. These compounds are present in a small group of plant species, among which, in particular, *polygonaceae* and *vitaceae.* From the former, and particularly from the dried roots of *polygonum cuspidatum,* a product used in traditional Chinese medicine is obtained, whose activity is actually attributed to the presence of resveratrol and its related compounds. From the vitaceae grapes and wine are obtained, which - thanks to the presence of resveratrol - are currently considered to be responsible for beneficial effects on cardiovascular diseases and arteriosclerosis when included in the diet in the proper amounts.

The anti-inflammatory activity of resveratrol is mainly due to the capacity of this molecule to interact with the prostaglandins H₂-synthesases (called COX-1 and COX-2) involved in prostaglandin synthesis. This activity is also correlated to cardioprotective activity and, partly, to chemoprotective activity. The results of many studies attribute these biological properties to the powerful antioxidant activity of resveratrol, and this activity is also ascribed to its capacity to modulate the lipid metabolism by protecting the low density lipoproteins (LDL), and particularly PUFA, from oxidation. Moreover, by protecting cell membrane lipids, resveratrol reduces the deleterious effects of oxidative stress on cells and tissues.

The family of compounds of natural origin having resveratrol as its main exponent has been the object of many studies aiming to identify the various compounds present in the plant extracts of spermatophyte plants containing them, and to determine their biological activity and possible use in the pharmacological field. Among the many documents of scientific and patent literature, for example, European patent EP 1292319 (Istituto di Medicina Sperimentale of CNR and Istituto Agrario of S. Michele *all'Adige),* one of the authors of which is the present inventor, covers a trimer of resveratrol, H-gnetine, and two tetramers of the same, r-viniferin and r-2-viniferin, as being new compounds isolated from the vine or polygonum extracts. The patent also covers the use of a broader group of these stilbenes and stilbenoids (dimers, trimers and tetramers of *cis*- and *trans*-resveratrol, as well as the relative glucosylated derivative, known with the name of piceide or resveratrol-3-O-β-mono-D-glucoside) for the treatment of colorectal carcinoma and melanoma. Similarly, European patent EP 1292320, of the same owners and authors, covers the use of the same broader group of compounds for the treatment of some immuno-deficiencies and autoimmune diseases.

Despite the vast literature available, the use of resveratrol and its related compounds in the ophthalmic field has not been studied in as great a depth. One document which can be related to this topic is US patent No. 6573299 (E. J.Petrus, assigned to Advanced Medical Instruments), which describes methods and compositions for the treatment of (physiological and pathological) ageing of the eye. This document, in fact, describes a copious set of biologically active agents useful as antioxidants, including resveratrol (but not featuring all its other related natural extractive compounds), that can be included together with a transdermic permeation promoter in a preparation with functions that are partly cosmetic and partly therapeutic, to be applied on eyelids. According to the aforesaid document, the proposed antioxidant preparation not only attenuates wrinkles and blemishes on eyelids, but also penetrates into the surrounding tissues, also treating the forming of cataract, glaucoma, diabetic retinopathy and macular degeneration.

Another prior art document devoted to resveratrol in the ophthalmic field is the publication by King et al. in Chemico-Biological Interactions of 2005 (King R.E., Kent K.D., Bomser J.A., Resveratrol reduces oxidation and proliferation of human retinal pigment epithelial cells via extracellular signal regulated kinase inhibition, Chemico-Biological Interactions, 151, 2005, 143-149), wherein it is demonstrated with *in vitro* trials that resveratrol can have beneficial effects on retinal pathologies associated to oxidative stress such as AMD and PVR. Here, too, the study only dealt with *trans*-resveratrol.

On the basis of this state of the art, the present invention thus aims to provide a preparation - preferably based on antioxidant agents of natural origin - for topical ophthalmic, oral or parenteral administration, that is both capable of preserving and restoring the structural and functional characteristics of ocular tissues in ophthalmic pathologies involving different eye structures, and namely of the cornea (such as dry eye, corneal erosions and lesions, outcomes of surgical operations like PRK and LASIK, and phacoemulsification), lens (such as cataract), retina (such as age-related macular degeneration, diabetic retinopathy) and optic nerve (such as glaucoma).

To this end, the present invention proposes the use, in the formulation proposed, of a glucosylated hydroxystilbene as active ingredient, and in particular piceide (or resveratrol-3-O-β-mono-D-glucoside, also known as polydatin). Also this compound, which can be represented, in the *trans-* form, by the structure of formula (I) 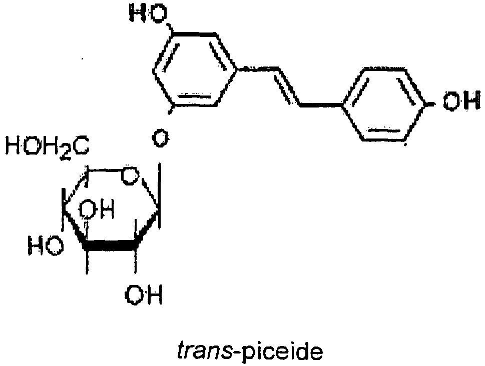 is found in abundance in spermatophyte plants of the *polygonaceae* and *vitaceae* families as well as in the *cyperaceae, gnetaceae* and *leguminosae.* In particular, in *vitis vinifera,* where - along with resveratrol - piceide is the most 3,5-dihydroxystilbene-4'-O-β-mono-D-glucoside (resveratroloside), 3',4',5-trihydroxystilbene-3-O-β-monoglucoside (astringin), 4'-hydroxystilbene-3,5-O-β-diglucoside,
5-hydroxystilbene-3,4'-O-β-diglucoside,
3-hydroxystilbene-4',5-O-β-diglucoside,
stilbene-3,4',5-O-β-triglucoside,
all in both *trans-* and *cis-* forms.

The present invention specifically provides the use of resveratrol-3-O-β-mono-D-glucoside (piceide) for the production of a pharmaceutical/nutritional preparation for the prevention and treatment of corneal haze formation and delayed corneal epithelial healing following refractive surgery and for the treatment of corneal erosions and lesions, wherein the said eye conditions involve an imbalance in the production of TGF-β in the affected site.

Preferably, the compound used in the pharmaceutical and/or nutritional preparations according to the present invention is selected from *trans-*resveratrol-3-O-β-mono-D-glucoside (trans-piceide) and *cis*-resveratrol-3-O-β-mono-D-glucoside (cis-piceide), which can also be represented, respectively, by the following two structural formulas, wherein the glucose ring is symbolically given as Glu-.

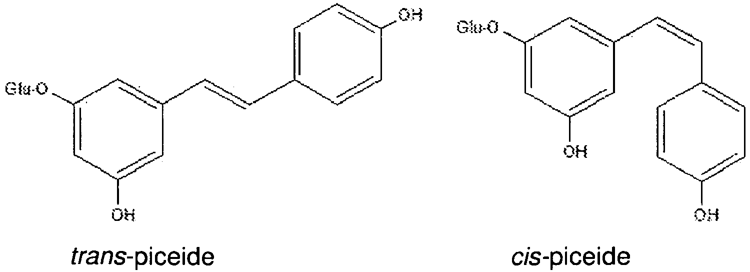

According to some preferred embodiments of the present invention, the said proposed pharmaceutical/nutritional preparation may be a preparation for topical ophthalmic administration, in particular, in the form of eye-drops or ophthalmic solution, cream or gel, or carried in macro-, micro- or nano- group consisting of:
resveratrol-3-O-β-mono-D-glucoside (piceide),
3,5-dihydroxystiibene-4'-O-β-mono-D-glucoside (resveratroloside),
3',4',5-trihydroxystilbene-3-O-β-monoglucoside (astringin),
4'-hydroxystilbene-3,5-O-β-diglucoside,
5-hydroxystilbene-3,4'-O-β-diglucoside,
3-hydroxystilbene-4',5-O-β-diglucoside,
stilbene-3,4',5-O-β-triglucoside,
all in both trans- and cis- forms.

Preferably, the compounds used in the pharmaceutical and/or nutritional preparations according to the present invention are selected from the group consisting of *trans*-resveratrol-3-O-β-mono-D-glucoside (trans-piceide) and *cis*-resveratrol-3-O-β-mono-D-glucoside (cis-piceide), which can also be represented, respectively, by the following two structural formulas, wherein the glucose ring is symbolically given as Glu-.

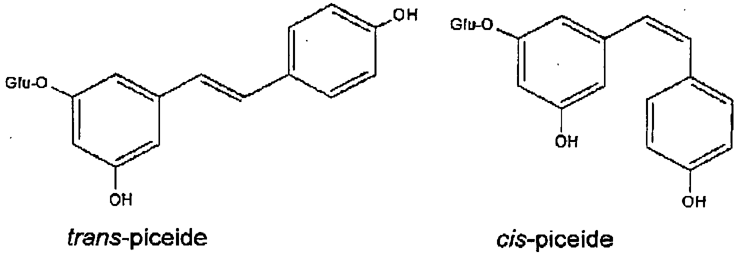

According to some preferred embodiments of the present invention, the said proposed pharmaceutical/nutritional preparation may be a preparation for topical ophthalmic administration, in particular, in the form of eye-drops or ophthalmic solution, cream or gel, or carried in macro-, micro- or nanoparticles, in liposomes or in macro- and micro-emulsions. Alternatively, the preparation proposed may be for oral administration, in particular, in the form of tablets, capsules, syrup, drinkable solution, granulate or preparations for sublingual administration, or it may be a product for parenteral administration, in particular, via intramuscular or intravenous injection.

The effects of the administration or intake of piceide and of other compounds according to the present invention on the single structures and/or pathologies can be intended for prevention or for treatment purposes, and may be due to different action mechanisms.

The pathological manifestations of the cornea are mainly due to inflammatory processes. As already observed, one of the causes of the onset of the aforesaid inflammatory processes is the stimulation - caused by an increase in ROS - of the release of inducible nitric-synthesase oxide (iNOS) by corneal cells, with the subsequent synthesis of NO, thus leading to inflammatory processes and cytotoxicity.

As will be illustrated in greater detail in the experimental section below, piceide - at non-toxic levels - blocks iNOS production and its activity is correlated to the dose. Comparing the dose/effect responses of piceide and resveratrol it is possible to highlight how, with equal doses, the former is surprisingly more effective than the latter in inhibiting iNOS production by fibroblasts of normal corneal origin and by fibroblasts of corneal origin immortalised by human Telomerase Reverse Transcriptase (hTERT), stimulated with interleukin-1 (IL-1). This different activity is probably due to the different chemical structure of the two molecules and to the consequent different biological characteristics, among which the greater bioavailability of piceide at the cell level is important. Another anti-inflammatory effect of piceide, linked to what has been said above, lies in its capacity to inhibit cyclooxygenase synthesis and thus the synthesis and release of pro-inflammatory prostaglandins.

The integrity of the eye surface and the repair of corneal lesions depends on a delicate balance between the proliferation, differentiation, migration and apoptosis of different kinds of cells (Klenkler B. & Sheardown H., Exp. Eye Res., 2004, 79, 677-688). These functions are regulated by different growth factors that can be produced by the corneal epithelium, stroma and endothelium. Even the tear film adjacent to the cornea and the aqueous humour are a source of growth factors. As is known, growth factors are a heterogeneous group of hydrosoluble peptides produced by different types of cells, and their action may be autocrine and/or paracrine, being capable of triggering or inhibiting the various cell functions. They act by bonding to their respective cell receptors - transmembrane glycoproteins belonging to the tyrosine kinase receptor (TKR) family or G-protein coupled receptors (GPCR). The effects of the various growth factors on a certain type of cell depend on various elements, such as the extracellular environment in which these factors come into contact with the cells, the growth factor concentration, the interaction between the various factors present according to their concentrations.

An important growth and regulating factor is transforming growth factor-β (TGF-β). This factor is synthesised by many kinds of cells, including the keratinocytes, activated macrophagi and fibroblasts, and has a great many important biological - and sometimes conflicting - effects on cell processes. TGF-β, for example, inhibits epithelial cell growth but causes mitosis in mesenchymal cells like fibroblasts. It has also been shown that TGF-β has important functions in corneal lesion repair processes. In fact, during repair there is an upregulation of TGF-β specific receptors on corneal epithelial cells, and it has been demonstrated that during this process the factor regulates the expression of some components of the extracellular matrix (ECM) and the anti-inflammatory response. Moreover, TGF-β regulates the transformation of keratocytes into migratory fibroblasts and into myofibroblasts, a population of cells that are essential in the cornea re-cicatrisation process.

The cornea repair process may be briefly summarised as follows:

12-24 hours after a lesion, there is the release of the TGF-β produced by the epithelial cells in the stroma, with the resulting stimulation of the stem cells. The quiescent keratocytes are thus activated to differentiate into migrant fibroblasts and subsequently into myofibroblasts, which are cells characterised by the expression within them of bunches of actin filaments of the isoform called α-SMA (α-smooth muscle actin) since it is only found on smooth muscle. As already noted, these cells play a fundamental role in repairing lesions because they form a meshwork of cells that can exert a mechanical force through a contractile type mechanism of a smooth muscle type, that acts giving rise to the contraction of the lesion. Moreover, as already described, TGF-β release stimulates the organisation of the matrix.

Still within a time frame of 12-24 hours after the lesion, in the cornea there is a flow of inflammatory cells, i.e. monocytes, macrophagi or even fibroblasts, coming from limbar vessels. In this case, too, TGF-β plays a fundamental role because it activates the inflammatory cells to reach the inflammation site.

Generally, weeks (or sometimes months) after the lesion and its subsequent repair, the cornea returns to a normal state, by eliminating the myofibroblasts and inflammatory cells, or thanks to the return to pre-lesion levels of the growth factors, thereby regaining its own normal optical characteristics. The success of this process depends very much on the correct combination of growth factors present, in particular, of TGF-β.

It is important to consider that, when this factor is present in the lesion site in appropriate concentrations and at the right time, it is an essential regulator of normal corneal functioning and of the repair mechanism. However, an imbalance of this factor can lead to pathological situations, such as an incomplete recovery of the lesion followed by a corneal pathology, with the associated loss in visual capacity. In fact, an excessive production of TGF-β during traumatic or infective conditions can lead to an excessive production of extracellular matrix, to an excessive infiltration of inflammatory cells (and thus to a pathological inflammatory reaction), or to a stimulation of pro-angiogenic cytokine (VEG, FGF) production, and thus to cornea neovascularization. These pathological actions of TGF-β lead to corneal oedema, and the haziness and loss of cornea optical quality. Moreover, the excessive stimulation of myofibroblast formation or their preservation for non-physiological time periods on the part of TGF-β leads to the aforesaid corneal haze. As already noted, it has also recently been demonstrated (Ivarsen A. et al., Br. J. Ophthalmol. 2003, 87, 1272-1278) that the fibrotic margin which forms for more or less long time periods following a LASIK operation on the cornea and which causes refractivity changes in it is due to the stimulation of TGF-β production, and thus to myofibroblast formation.

As reported in detail in the experimental section below, piceide has - unlike resveratrol - unexpectedly proven to be capable of modulating the capacity of TGF-β to cause fibroblast differentiation into myofibroblasts. In fact, when added to cultures of fibroblasts of normal corneal origin or to hTERT fibroblasts stimulated with a *sub-optimal* concentration of TGF-β, incapable of causing the differentiation of fibroblasts into myofibroblasts, piceide has been found to increase growth factor activity, thereby restoring its biological capacity, assessed by determining α-SMA expression. On the other hand, when piceide is added to cultures of normal fibroblasts or hTERT fibroblasts stimulated with an above-optimal concentration of TGF-β, it causes a decrease in α-SMA expression, thus demonstrating it has the ability to modulate the growth factor effect. Moreover, at non-cytotoxic or cytostatic doses, piceide does not have significant activity on either type of cell.

It is evident that this piceide activity has an important therapeutic effect because it stimulates proper and physiological cornea repair in those cases where there is an ascertained delay in reparative processes, but it prevents the formation of haze in those, not infrequent, cases - for example, in refractive surgery - in which there is an abnormal growth and/or permanence of myofibroblasts in the lesion site.

It must be noted that this activity described within the present invention contrasts with what has been published for myofibroblasts of hepatic origin, on which piceide - unlike resveratrol - did not show any significant effects (Godichaud S. et al., Deactivation of cultured human liver myofibroblasts by trans-resveratrol, a grapevine-derived polyphenol, Hepatology, 2000, 31, 922-931). According to the cited reference, *trans*-resveratrol causes a reduction of the α-SMA expression and has proven to be able to deactivate human liver myofibroblasts, which are considered to be the ones most responsible for human liver fibrosis and liver tumours. On the other hand, neither *trans-*piceide nor another hydroxystilbene - *trans*-piceatannol *(trans-*3,4',3',5-tetrahydroxystilbene) - have shown any effect on liver myofibroblasts. This difference, found for the first time within the frame of the present invention, is probably due to the different cell conveyance mechanism between the two substances, and to the different type of cells concerned.

The free radical scavenger activity of piceide underlies its therapeutic effect as regards corneal damage caused by phacoemulsification, and this activity also underlies its capacity to prevent cataract development by contrasting the effects of repeated oxidative damage caused by light rays during individual lifetime. In fact, lens protein oxidation is an important pathophysiological factor of cataract, and thus the powerful antioxidant action of piceide is a particularly efficient protection in this regard.

A weakening of the antioxidant defence system has also been found with certain patients with glaucoma, and particularly their local GSH levels, and thus piceide use can also be considered a substitute preventive therapy.

As already reported, even in macular degeneration the free radical damage due to exposure to light plays an important pathogenetic role. Endogenous antioxidants are normally present at retinal level and their reduction is assumed to contribute to the onset of the pathology. Prolonged treatments with antioxidants have proven to be useful in delaying or even preventing the onset of the pathology. In this case, too, piceide use, thanks to its antioxidant properties, can be regarded as a preventive or curative treatment, in that it hinders or delays the onset of the pathology.

Finally, since TGF-β is a powerful inductor of VEGF (Vascular Endothelial Growth Factor) secretion and since, as already noted, this factor is important in the ethiology of choroid neovascularization (CNV) and AMD neovascularization, piceide - thanks to its capacity to modulate TGF-β activity - is an effective therapeutic agent also for these two ophthalmic pathologies.

The active ingredient(s) in the preparation according to the present invention are preferably present in the formulation in a total quantity ranging from 0.001 % to 10% in weight. As noted, the said active ingredient is resveratrol-3-O-β-mono-D-glucoside (piceide), and preferably trans-piceide.

In particular, the preparation according to the present invention can be in the form of eye-drops, and may contain from 0.001% to 10% weight of trans-piceide in an aqueous solution. According to certain embodiments of the formulation, the preparation can also include hyaluronic acid, in particular, in tenors ranging between 0.01% and 2% by weight.

Other possible ingredients to be added in a formulation according to the present invention, instead of hyaluronic acid, are, for example, various cellulose derivatives (such as hydroxypropylmethylcellulose and carboxymethylcellulose), other polysaccharides, polyvinyl alcohol, phospholipid-based microemulsions and polyvinylpyrrolidone (PVP, povidone).

Obviously, a liquid formulation based on the aforesaid active ingredients may contain, as excipients, various other ingredients well-known in the pharmaceutical field, such as pH regulating agents and buffers, preserving agents, chelating agents and one or more tonicity adjusting agents, that can give the solution the desired osmolarity value. Since the proposed ophthalmic solution should be isotonic or slightly hypotonic with respect to lachrymal fluid, these tonicity-adjusting agents will be present in the preparation in suitable amounts to provide a solution with an osmolarity between 170 and 350 mOsm/L.

In other embodiments of the present invention, the ophthalmic preparation based on piceide, and preferably trans-piceide, can be in a gel or cream form. In this case, the preparation can contain from 0.001 % to 1 % by weight of trans-piceide.

As already noted, the use of the active ingredient proposed by the present invention is also possible in a suitable form for oral administraadministration, such as in the form of capsules containing from 0.1 mg to 500 mg of trans-piceide per capsule. In certain cases it may be advantageous to formulate the preparation with the addition of polyunsaturated fatty acids, preferably in quantities ranging from 50 mg to 5000 mg per capsule.

Finally, when the preparation of antioxidant and anti-inflammatory activity, according to the present invention, is for parenteral administration, in particular via intramuscular or intravenous injection, a convenient solution is that of a vial for intramuscular injection, containing from 0.1 mg to 500 mg of trans-piceide per vial.

The specific characteristics of the present invention, as well as its advantages and relative operative modalities, will be more evident with reference to a detailed description presented merely for exemplificative purposes below, together with the results of the experiments carried out on it and the data for comparison with the prior art. Some experimental results are also illustrated in the attached figures, wherein:
Figure 1 shows the percentage trend of iNOS-positive cornea hTERT fibroblasts in the case of their pre-incubation with trans-resveratrol and with trans-piceide;
Figure 2 shows the effect of piceide on the differentiation of corneal hTERT fibroblasts stimulated with sub-optimal doses of TGF-β, assessed by examining whether α-SMA is present;
Figure 3 shows the effect of piceide on the differentiation of corneal hTERT fibroblasts stimulated with optimal doses of TGF-β, assessed by examining whether α-SMA is present; and
Figure 4 shows the effect of piceide on the differentiation of corneal hTERT fibroblasts stimulated with above-optimal doses of TGF-β, still assessed by examining whether α-SMA is present.

Some specific embodiments of the preparations for the therapy and prevention of various ophthalmic conditions according to the invention are reported below merely for exemplificative purposes.

### EXAMPLE 1

### Eye-drop preparation for dry eye

The composition of a piceide-based collyrium to be used for topical administration in the eye as a tear fluid substitute is reported below.

| **Component** | **Concentration (%)** |
|---|---|
| piceide | 0.10 |
| hyaluronic acid | 0.15 |
| PEG 8000 | 0.50 |
| sodium bisulphate (NaHS0₃) | 0.075 |
| benzalkonium chloride | 0.005 |
| EDTA | 0.125 |
| phosphate buffer | q.s. to pH 6.0 - 7.5 |
| NaCl | q.s. to 270 mOsm/L |

The preparation, which must be used as ordinary artificial tears in a dosage of 1-2 drops per eye between 1 and 4 times a day, is able to alleviate the dry eye sensation, thanks to the combined presence of piceide and hyalurinic acid, formulated with suitable excipients, and also has an antioxidant and anti-inflammatory activity.

### EXAMPLE 2

### Eye-drop preparation for dry eye

As an alternative to the preceding preparation, here follows another eye-drop preparation useful as artificial tear fluid based on piceide, in this case to be packaged in monodose flasks due to the absence of any preservative.

| **Component** | **Concentration (%)** |
|---|---|
| piceide | 0.10 |
| phospholipid microemulsion | q.s. to 100 |

Since the aforesaid microemulsion is already stabilised, no other ingredients are necessary.

The doses and administration methods of the preparation are the same as the ones in the previous example.

### EXAMPLE 3

### Gel preparation for dry eye

In the most serious cases of dry eye, it may be more appropriate to employ a piceide-based gel preparation for use as a tear fluid substitute, with the following indicative composition.

| **Component** | **Concentration (%)** |
|---|---|
| piceide | 0.10 |
| carbopol | 0.20 |
| sorbitol | 4.00 |
| cetrimide | 0.01 |
| NaOH | q.s. to pH 7.2 |

The gel must be applied at the doses and frequency given for the eye-drops of Example 1.

### EXAMPLE 4

### Cream preparation for dry eye

Still in the most serious cases of dry eye, a form can be used wherein the antioxidant and anti-inflammatory agent is in a cream. An exemplificative formulation is given below

| **Component** | **Concentration (%)** |
|---|---|
| piceide | 0.10 |
| liquid paraffin | 20.0 |
| lanolin | 10.0 |
| vaseline | q.s. to 100 ml |

In this case the preparation must be applied once a day, in the evening, for night treatment, since its particular thickness may blur vision.

### EXAMPLE 5

### Capsule preparation for maculopathies

A product for oral use that employs the antioxidant and anti-inflammatory properties of piceide, in a combination with other ingredients of already experimented protective and antioxidant properties, and also with a series of olygoelements useful in the case of degenerative and ageing pathologies, can be of the following composition.

| | **Component** | **Concentration (per capsule)** |
|---|---|---|
| piceide | | 10.0 mg |
| PUFA (polyunsaturated fatty acids) | | 500 mg |
| | Omega-3 (DHA, EPA) | |
| | α-linoleic and γ-linoleic acids | |
| | Omega-6 | |
| | linolenic acid | |
| lipoic acid | | 300 mg |
| L-glutathione | | 1500 mg |
| selenium | | 0.04 mg |
| copper | | 0.60 mg |
| zinc | | 15.0 mg |
| lutein | | 6.0 mg |

For a proper protective action, the preparation must be taken in doses ranging between 1 and 4 capsules per day.

### EXAMPLE 6

### Capsule preparation for dry eye

Another product for oral administration which, unlike the one of Example 5, is indicated as an anti-inflammatory and antioxidant for dry eye, can have the following composition.

| **Component** | | **Concentration (per capsule)** |
|---|---|---|
| piceide | | 10.0 mg |
| PUFA (polyusaturated fatty acids) | | 500 mg |
| | Omega-3 (DHA, EPA) | |
| | α-linoleic and γ-linoleic acids | |
| | Omega-6 | |
| | linolenic acid | |
| N-acetylcysteine (NAC) | | 600 mg |
| vitamin C | | 750 mg |

The dosage is the same as the one given in Example 5.

### EXAMPLE 7

### Capsule preparation for vascular occlusions and maculopathies

A product according to the present invention for oral administration, particularly recommended for the prevention of vascular occlusions and in the treatment of age-related dry or wet maculopathies, can have the following composition.

| **Component** | **Concentration (per capsule)** |
|---|---|
| piceide | 10,0 mg |
| betain | 1000 mg |
| folic acid | 0.40 mg |
| vitamin B₁₂ | 0.0024 mg |
| vitamin B₆ | 1.70 mg |

The combination of the aforesaid products, besides having an anti-inflammatory and antioxidant effect, contrasts the formation of homocystein, a natural catabolyte whose build-up in the body can lead to heart and vascular problems.

In this case, too, the product must be taken in doses ranging from 1 to 4 capsules per day.

### EXAMPLE 8

### Injectable preparation for dry eye

When the conditions linked to the ophthalmic pathology are such to justify it, the piceide-based antioxidant and anti-inflammatory preparation according to the present invention may also envisage administration by means of intramuscular injection, in which case the product can have the following composition.

| **Component** | **Concentration (per capsule)** |
|---|---|
| piceide | 10.0 mg |
| water for injectables | q.s. to 5 ml |

In this case, the dosage limits recommended range from 1 to 4 phials per day.

### Effects of piceide on iNOS production by hTERT fibroblasts stimulated with cytokines

### Study aim

The study aimed at determining the effects of treatment with piceide and resveratrol on the inducible nitric-synthesase oxide (iNOS) by corneal fibroblasts immortalised with human Telomerase Reverse Transcriptase (hTERT fibroblasts), stimulated with interferon-γ (IFN-γ) and interleukine-1 (IL-1).

### Method

### Cells and treatment

Some hTERT fibroblasts were maintained in a DMEM culture medium. They were subsequently re-suspended at a concentration of 1x10⁶ and placed on 24 mm-well plates. The cells were incubated at a temperature of 37°C with piceide or resveratrol at doses of 1.25 - 2.5 - 5 µg/ml. After 15 minutes the following were added to the wells containing the cells: IL-1 at a concentration of 3 ng/ml, and IFN-γ at a concentration of 250 U/ml. After a further 48 h of incubation, the fibroblasts were washed and re-suspended in PBS at a concentration of 1x10⁶, in order to determine the iNOS-positive cells by means of flow cytometry.

### Flow cytometry

The cells treated in the aforesaid way were permeabilised by treatment with 70% ethanol and then labelled with 5 µg/ml of a human anti-iNOS antibody labelled with FITC. After; colouring, the cells were fixed in paraformaldehyde and analysed by means of a FACScan cytofluorimeter.

### Results and conclusions

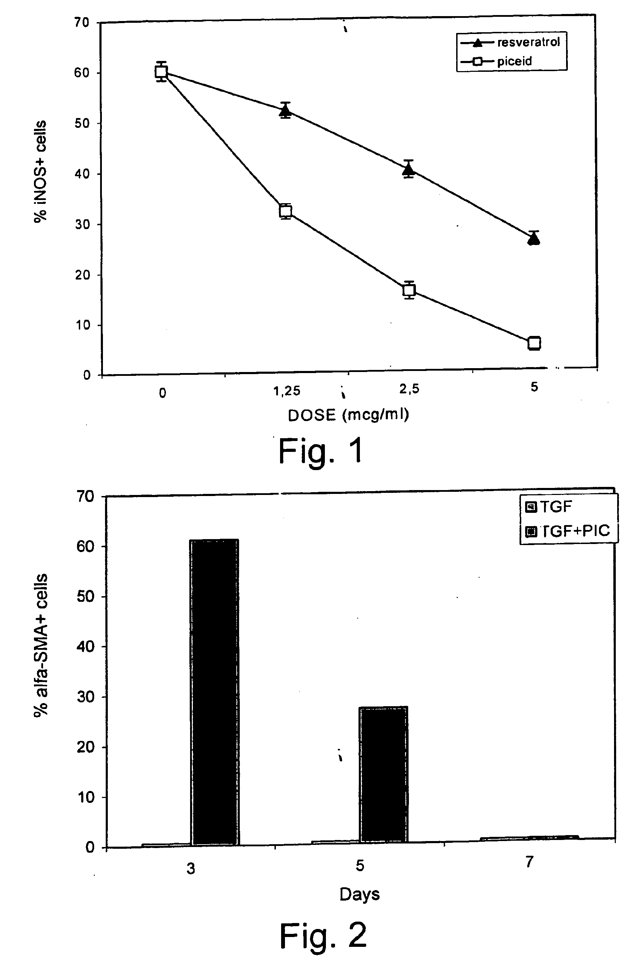

As illustrated graphically in Figure 1, the results show that stimulation with IL-1+ IFN-γ causes iNOS production in approx. 60% of the hTERT fibroblasts. Pre-incubation with piceide caused a dose-dependent decrease in the number of iNOS-positive fibroblasts. This decrease is greater compared to the one obtained with a pre-incubation with resveratrol. The differences between piceide and resveratrol are statistically significant at each point along the experimental curve.

At the doses examined in the present study, both compounds resulted non-toxic (data not shown).

From the aforesaid results; it is possible to conclude that piceide causes a significant decrease in the percentage of fibroblasts capable of producing iNOS after cytokine stimulation. This activity indicates that the compound has anti-inflammatory properties for human cells of corneal origin. The aforesaid results also indicate that the effectiveness of piceide is statistically greater than that of resveratrol.

### Effects of piceide on fibroblast differentiation

### Study aim

The study aimed at determining the effects of piceide treatment on the differentiation of corneal fibroblasts immortalised with human inverse telomerase transcriptase (hTERT fibroblasts) stimulated with sub-optimal, optimal or above-optimal doses of TGF-β. Fibroblast differentiation into myofibroblasts was determined by examining whether there was any α-smooth muscle actin (α-SMA).

### Method

### Cells and treatment

Some hTERT fibroblasts maintained in a DMEM culture were re-suspended at a concentration of 1x10⁶ and placed on 24mm-well plates. The cells were incubated at a temperature of 37°C with piceide or resveratrol at doses of 5 µg/ml. After 24 h the cells were stimulated with optimal (1ng/ml), sub-optimal (0.1 ng/ml) and above-optimum (10 ng ml) doses of TGF-β. After 3, 5 and 10 days of incubation, the fibroblasts were washed and re-suspended in PBS at a concentration of 1x10⁶, in order to determine the α-SMA-positive cells by means of flow cytometry.

### Flow cytometry

The cells treated in the aforesaid way were permeabilised by treatment with 70% ethanol and then labelled with 5µg/ml of a human anti-α-SMA antibody labelled with FITC. After colouring, the cells were fixed in paraformaldehyde and analysed by a means of a FACScan cytofluorimeter.

### Results and conclusions

As illustrated graphically in Figure 2, the experimental results show that adding 5 µg/ml piceide·to the cultures stimulated with the sub-optimal dose (0.1 ng/ml) of TGF-β determines, after 3 days of incubation, a statistically significant increase - compared to the non-treated ones - of the percentage of cells capable of expressing α-SMA (i.e. myofibroblasts). This increase decreased, although still remaining statistically significant, by the 5^{th} day, while by the 10^{th} day there were no longer any significant percentages of cells able to express α-SMA.

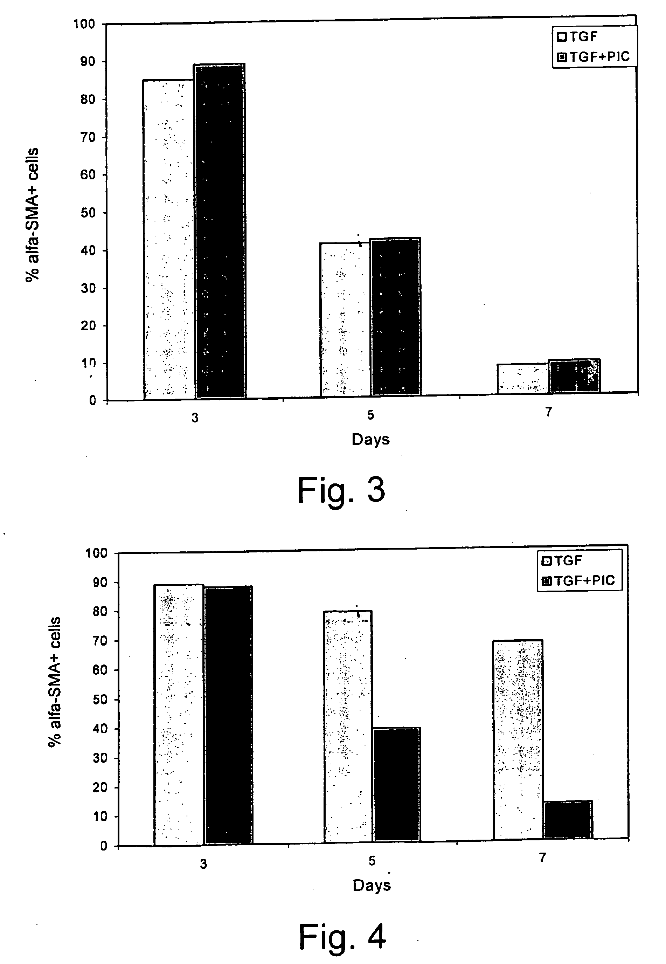

On the other hand, as shown in Figure 3, when 5 µg/ml piceide was added to the cultures stimulated with an optimal dose (1 ng/ml) of TGF-β it did not lead to any significant percentage variations of α-SMA-positive cells in all the three time intervals considered.

Stimulation of the cultures with TGF-β at an above-optimal dose of 10 ng/ml led to an increase in the number of myofibroblasts (α-SMA+ cells), as shown in Figure 4. This increase remained significant throughout the 10 days examined. The addition of 5 µg/ml piceide led to a percentage decrease in fibroblasts which was already significant by the 5^{th} day, and by the 10^{th} day brought the number of fibroblasts back to physiological conditions, with percentage values of α-SMA+ cells that were not statistically different from the ones recorded at the same time intervals with the optimal dose.

When 5 µg/ml resveratrol was added to the cultures stimulated with the sub-optimal, optimal and above-optimal dose of TGF-β, it did not lead to any statistically significant variation in the percentage of α-SMA+ cells.

In conclusion, it is evident that piceide has demonstrated having the capacity to modulate the effect of TGF-β in bringing about the differentiation of fibroblasts into myofibroblasts. Piceide has proven itself capable of both increasing the capacity of TGF-β, at a sub-optimal dose, to cause the differentiation into myofibroblasts, and also to restore to normal conditions the number of myofibroblasts, which had remained high on the 10^{th} day after stimulation with an above-optimal dose of TGF-β. This effect shows the capability of piceide to modulate and regularise myofibroblast production, thereby assuring a proper repair of the corneal lesion and a better preservation of the optical qualities of the cornea. At the doses assayed, piceide does not appear toxic for this cell line.

The results of the experimentation carried out also indicate that resveratrol does not show a similar effectiveness in modulating TGF-β activity.

From the above presentation, it appears evident that, thanks to its scavenger activity on free radicals, piceide is able to prevent oxidative damage, however caused, to the cornea and can treat the inflammatory process should this arise.

## Claims

1. Use of resveratrol-3-O-β-mono-D-glucoside (piceide) for the production of a pharmaceutical/nutritional preparation for the prevention and treatment of corneal haze formation and delayed corneal epithelial healing following refractive surgery and for the treatment of corneal erosions and lesions, wherein the said eye conditions involve an imbalance in the production of TGF-β in the affected site.

2. Use according to claim 1, wherein the said resveratrol-3-O-β-mono-D-glucoside is chosen from *trans*-resveratrol-3-O-β-mono-D-glucoside and *cis*-resveratrol-3-O-β-mono-D-glucoside.

3. Use according to claim 2, wherein the said pharmaceutical/nutritional preparation contains *trans*-resveratrol-3-O-β-mono-D-glucoside as active ingredient.

4. Use according to any one of claims 1-3, wherein the said preparation is a product in a form suitable for topical ophthalmic administration, oral administration or parenteral administration.

5. Use according to any one of claims 1-4, wherein the said-piceide is present in the said preparation in a total amount ranging from 0.001 % to 10% by weight.

6. Use according to claim 5, wherein the said piceide is *trans-*resveratrol-3-O-β-mono-D-glucoside, i.e. *trans*-piceide.

7. Use according to claim 6, wherein the said preparation is in a form suitable for oral administration.

8. Use according to claim 7, wherein the said preparation is in the form of capsules containing from 0.1 mg to 500 mg of *trans*-piceide per capsule.

9. Use according to claim 8, wherein the said preparation also contains from 50 mg to 5000 mg of polyunsaturated fatty acids per capsule.

10. Use according to claim 6, wherein the said preparation is in a form suitable for parenteral administration.

11. Use according to claim 6, wherein the said preparation is in the form of a vial for intramuscular injection and containing from 0.1 mg to 500 mg of *trans-*piceide per vial.

## Patentansprüche

1. Verwendung von Resveratrol-3-O-**β**-mono-D-glucosid (Piceid) zur Herstellung eines pharmazeutischen Präparats bzw. Ernährungspräparats zur Prävention und Behandlung von Hornhauttrübungsbildung und verzögerter Hornhautepithelheilung nach einem Eingriff zur Korrektur von Refraktionsfehlern, und zur Behandlung von Hornhauterosionen und -läsionen, wobei die genannten Augenerkrankungen ein Ungleichgewicht bei der Produktion von TGF-**β** an der betroffenen Stelle beinhalten.

2. Verwendung nach Anspruch 1, wobei das Resveratrol-3-O-**β**-mono-D-glucosid aus *trans*-Resveratrol-3-O-**β**-mono-D-glucosid und cis-Resveratrol-3-O-**β**-mono-D-glucosid ausgewählt ist.

3. Verwendung nach Anspruch 2, wobei das pharmazeutische Präparat bzw. Ernährungspräparat *trans*-Resveratrol-3-O-**β**-mono-D-glucosid als aktiven Bestandteil enthält.

4. Verwendung nach irgendeinem der Ansprüche 1-3, wobei das Präparat ein Produkt ist, welches in einer für die topische ophthalmische Verabreichung, orale Verabreichung oder parenterale Verabreichung geeigneten Form vorliegt.

5. Verwendung nach irgendeinem der Ansprüche 1-4, wobei das Piceid in dem Präparat in einer Gesamtmenge im Bereich von 0,001 Gew.-% bis 10 Gew.-% vorliegt.

6. Verwendung nach Anspruch 5, wobei das Piceid *trans-*Resveratrol-3-O-**β**-mono-D-glucosid, d.h. *trans*-Piceid, ist.

7. Verwendung nach Anspruch 6, wobei das Präparat in einer für die orale Verabreichung geeigneten Form vorliegt.

8. Verwendung nach Anspruch 7, wobei das Präparat in Form von Kapseln, die 0,1 mg bis 500 mg trans-Piceid pro Kapsel enthalten, vorliegt.

9. Verwendung nach Anspruch 8, wobei das Präparat ferner 50 mg bis 5000 mg mehrfach ungesättigter Fettsäuren pro Kapsel enthält.

10. Verwendung nach Anspruch 6, wobei das Präparat in einer für die parenterale Verabreichung geeigneten Form vorliegt.

11. Verwendung nach Anspruch 6, wobei das Präparat in Form einer Ampulle für die intramuskuläre Injektion vorliegt und 0,1 mg bis 500 mg trans-Piceid pro Ampulle enthält.

## Revendications

1. Utilisation de resvératrol-3-O-β-mono-D-glucoside (picéide) pour la production d'une préparation pharmaceutique/nutritive destinée à la prévention et au traitement de la formation d'une opacité cornéenne et d'un retard de cicatrisation de l'épithélium cornéen à la suite d'une chirurgie réfractive et au traitement des érosions et des lésions cornéennes, dans laquelle lesdites affections oculaires impliquent un déséquilibre dans la production de TGF-β sur le site affecté.

2. Utilisation selon la revendication 1, dans laquelle ledit resvératrol-3-O-β-mono-D-glucoside est choisi parmi le trans-resvératrol-3-O-β-mono-D-glucoside et le cis-resvératrol-3-O-β-mono-D-glucoside.

3. Utilisation selon la revendication 2, dans laquelle ladite préparation pharmaceutique/nutritive contient du trans-resvératrol-3-O-β-mono-D-glucoside en tant que principe actif.

4. Utilisation selon l'une quelconque des revendications 1 à 3, dans laquelle ladite préparation est un produit sous une forme appropriée pour une administration ophtalmique topique, une administration orale ou une administration parentérale.

5. Utilisation selon l'une quelconque des revendications 1 à 4, dans laquelle ledit picéide est présent dans ladite préparation en une quantité totale allant de 0,001 % à 10 % en poids.

6. Utilisation selon la revendication 5, dans laquelle ledit picéide est le trans-resvératrol-3-O-β-mono-D-glucoside, c'est-à-dire le trans-picéide.

7. Utilisation selon la revendication 6, dans laquelle ladite préparation se présente sous une forme appropriée pour une administration orale.

8. Utilisation selon la revendication 7, dans laquelle ladite préparation se présente sous la forme de gélules contenant de 0,1 mg à 500 mg de trans-picéide par gélule.

9. Utilisation selon la revendication 8, dans laquelle ladite préparation contient également de 50 mg à 5000 mg d'acides gras polyinsaturés par gélule.

10. Utilisation selon la revendication 6, dans laquelle ladite préparation se présente sous une forme appropriée pour une administration parentérale.

11. Utilisation selon la revendication 6, dans laquelle ladite préparation se présente sous la forme d'un flacon pour injection intramusculaire et contenant de 0,1 mg à 500 mg de trans-picéide par flacon.
